# EUROPÄISCHE PATENTSCHRIFT

(11) **EP 3 509 683 B1**
(45) Veröffentlichungstag und Bekanntmachung des Hinweises auf die Patenterteilung: **24.01.2024**
(21) Anmeldenummer: 17790957.9
(22) Anmeldetag: 09.09.2017
(51) Int. Cl.: A61M 16/10, A61M 16/12, A61M 19/00, A61F 7/00, A61F 7/12

(54) **TRANSPORTABLE VORRICHTUNG UND SYSTEM ZUM ABSENKEN DER KÖRPERTEMPERATUR EINES SÄUGETIERS ÜBER DIE ATEMWEGE, INSBESONDERE EINES MENSCHEN, MITTELS EINES GEKÜHLTEN, SAUERSTOFFHALTIGEN GASSTROMS**
TRANSPORTABLE DEVICE AND SYSTEM FOR LOWERING THE BODY TEMPERATURE OF A MAMMAL OVER THE AIRWAYS, IN PARTICULAR OF A HUMAN, BY MEANS OF A COOLED, OXYGEN-CONTAINING GAS FLOW
DISPOSITIF TRANSPORTABLE ET SYSTÈME POUR ABAISSER LA TEMPÉRATURE CORPORELLE D'UN MAMMIFÈRE À TRAVERS LES VOIES RESPIRATOIRES, EN PARTICULIER D'UN ÊTRE HUMAIN, AU MOYEN D'UN FLUX GAZEUX REFROIDI CONTENANT DE L'OXYGÈNE

(30) Priorität: 09.09.2016 LU 93200
(43) Veröffentlichungstag der Anmeldung: 17.07.2019
(73) Patentinhaber: Temme, Fabian, 33330 Gütersloh (DE)
(72) Erfinder: Temme, Fabian, 33330 Gütersloh (DE)
(74) Vertreter: Von Rohr Patentanwälte Partnerschaft mbB
(86) Internationale Anmeldenummer: PCT/EP2017/001065
(87) Internationale Veröffentlichungsnummer: WO 2018/046128

(56) Entgegenhaltungen:
- WO-A1-2013/090730
- WO-A2-03/047603
- WO-A2-2004/082729
- US-A1- 2008 262 377
- US-A1- 2010 108 063
- US-A1- 2012 031 405
- US-A1- 2014 338 668
- US-A1- 2015 151 073

## Beschreibung

Die Erfindung betrifft eine transportable Vorrichtung zur Bereitstellung eines gekühlten, sauerstoffhaltigen Gasstroms für die Zuführung zu einem Körper eines Säugetiers über die Atemwege, insbesondere eines Menschen, um die Körpertemperatur abzusenken. Darüber hinaus betrifft die Erfindung ein System mit wenigstens einer Vorrichtung der vorgenannten Art.

Es ist bekannt, dass jährlich eine Vielzahl von Menschen einen Schlaganfall erleidet. Ein Schlaganfall, auch Hirn- oder Gehirnschlag genannt, ist die Folge eines verminderten Blutflusses zum Gehirn des Betroffenen, wodurch Gehirnzellen absterben können. Dabei lassen sich zwei Arten von Schlaganfällen unterscheiden. Ein ischämischer Schlaganfall wird durch einen verminderten Blutfluss ausgelöst. Von einem hämorrhagischen Schlaganfall spricht man, wenn die Durchblutung aufgrund von Ausblutungen aus beschädigten oder geplatzten Adern oder beschädigtem Gewebe verringert und/oder unterbrochen ist.

Durch beide Arten von Schlaganfällen kann die Leistung des Gehirns oder Teilen des Gehirns eingeschränkt werden. Symptome eines Schlaganfalls können Bewegungseinschränkungen oder ein Taubheitsgefühl auf einer Körperseite sein. Des Weiteren kann es den Betroffenen Probleme bereiten, zu sprechen oder die Hörfunktion kann eingeschränkt sein. Das Sehvermögen kann eingeschränkt sein und der Betroffene kann unter einem Schwindelgefühl leiden. Halten die Symptome weniger als ein oder zwei Stunden an, spricht man von einer vorübergehenden Durchblutungsstörung.

Die Symptome eines Schlaganfalls können dauerhaft anhalten. Die Langzeitfolgen können Lungenentzündungen und einen Verlust der Harnkontrolle beinhalten.

Es ist daher von großer Bedeutung, im Falle eines Schlaganfalls diese Symptome zu vermeiden oder wenigstens zu verringern, d.h. die Organe, wie beispielsweise das Gehirn und Herz, zu schützen um das Leben des Patienten zu retten und gleichzeitig die Langzeitfolgen zu vermeiden oder zu minimieren. Die Sauerstoffsättigung des Gewebes ist zur Erreichung dieser Ziele von großer Bedeutung. Nach aktuellen medizinischen Leitlinien (DEGAM Leitlinie 8, 2012) soll im Falle eines Schlaganfalls zuerst sichergestellt werden, dass die Atemwege des Patienten freigehalten werden. Als nächstes soll der Patient in eine bequeme Position überführt und mit Sauerstoff versorgt werden und es wird ein intravenöser Zugang gelegt, wobei Blutdruck und der Blutzuckerspiegel gemessen werden sollen.

Des Weiteren ist es bekannt, dass ein Kühlen des Gewebes den Sauerstoffverbrauch vorteilhaft herabsenkt.

In der US 5,261,339 A sowie in der US 5,957,964 A ist das Kühlen vom Gewebe eines Patienten beschrieben, einerseits durch einen Helm, der den Kopf extern kühlt, andererseits durch die Verwendung einer vorgekühlten Infusion.

Aus der US 7,201,163 B2 und der DE 10 2007 019 616 A1 ist bekannt, dass ein Herabsetzen der Körpertemperatur auch über die Atemwege erfolgen kann. Während die US 7,201,163 B2 die Zufuhr feuchter, vernebelter Luft offenbart, beschreibt die DE 10 2007 019 616 A1 die Zuführung eines verflüssigten Gases als Kühlmedium in die Atemluft.

Trotz der bekannten Therapieformen lassen sich die Langzeitfolgen von Patienten nicht immer verhindern, da beispielsweise die Behandlung erst im Krankenhaus erfolgen kann und der Transport in entsprechenden Einrichtungen entscheidende Zeit beansprucht.

Die WO 2011/115964 A1 offenbart in diesem Zusammenhang ein System zur therapeutischen Kühlung von Bereichen des Körpers, wie beispielsweise des Gehirns. Dabei wird ein Gas beim Austritt aus einem Sauerstoffbehälter adiabat gekühlt und in diesem Zustand den Atemwegen eines Patienten zugeführt.

Die US 2008/0262377 A1 offenbart ein Verfahren und eine Vorrichtung zur Steuerung der Körpertemperatur eines Patienten. Ein Atemgasgemisch wird dabei mittels einer Heiz- und/oder Kühleinrichtung erwärmt oder abgekühlt. Als Gas kann Luft oder ein spezielles Atemgemisch, wie beispielsweise ein Gemisch aus Helium und Sauerstoff, verwendet werden. Um die Wärmeübertragungsrate weiter zu erhöhen, kann das System einen Eispartikelgenerator zum Einführen von Feineispartikeln in den Fluss des Atemgases umfassen.

US 2012/0031405 A1 beschreibt ein Hirnkühlsystem, das ein Gasabgabesystem und eine Kühlvorrichtung umfasst. Das Gasabgabesystem kann dabei einen gewünschten Druck und eine Strömungsrate für Gase erzeugen, die von einem Patienten eingeatmet werden sollen. Die Zuführung der gekühlten Atemgase zum Patienten kann bei erhöhtem Sauerstoffanteil und einem erhöhten Luftdruck, der einen normalen physiologischen Luftdruck übersteigt, erfolgen.

Die WO 2013/079227 A1 beschreibt eine Vorrichtung, mit der das Gehirn eines Patienten, der an einem Herz-Kreislauf-Notfall leidet, intranasal gekühlt werden kann. Die Vorrichtung umfasst eine Kanüle zur Einführung in die Atemwege des Patienten, wobei ein Gas, das sich adiabatisch beim Entspannen abgekühlt hat, dem Patienten zugeführt wird.

Die US 2010/0108063 A1 offenbart eine Gasmaske und eine zugeordnete Beatmungsvorrichtung, wobei ein Wärmetauscher vorgesehen ist, der mittels eines Verdunstungsmittels gekühlt wird.

Aus der US 2015/0151073 A1 geht ein System und ein Verfahren zur Regulierung der Körpertemperatur eines Nutzers hervor, wobei gekühlte oder erwärmte Luft dem Nutzer manuell oder mittels eines Ventilators über eine Inhalationsleitung und über eine Atemmaske zugeführt wird.

Die US 2014/0338668 A1 offenbart eine Atemmaske zur Regulierung der Körpertemperatur eines Patienten unter Verwendung eines Atemgases, wobei ein Regler zur Bereitstellung des Atemgases sowie eine Gasleitung zur Ableitung des Atemgases vorgesehen sind.

Der Erfindung liegt die Aufgabe zugrunde, eine Vorrichtung und ein System jeweils der eingangs genannten Art zur Verfügung zu stellen, die in effektiver und einfacher Weise die Bereitstellung eines gekühlten, sauerstoffhaltigen Gasstroms zur therapeutischen Behandlung eines menschlichen oder tierischen Patienten, insbesondere zur Verringerung und/oder Verbesserung der Langzeitauswirkungen eines Schlaganfalls, ermöglichen.

Die vorgenannte Aufgabe wird durch eine Vorrichtung mit den Merkmalen von Anspruch 1 sowie ein System mit den Merkmalen von Anspruch 9 gelöst. Vorteilhafte Ausgestaltungen der Erfindung sind Gegenstand der Unteransprüche.

Erfindungsgemäß wird eine transportable Vorrichtung zur Bereitstellung eines gekühlten, sauerstoffhaltigen Gasstroms für die Zuführung zu einem Körper eines Säugetiers über die Atemwege, insbesondere eines Menschen, vorgeschlagen, um die Körpertemperatur abzusenken. Die erfindungsgemäße Vorrichtung weist eine wenigstens einen Fluidspeicher aufweisenden Speichereinrichtung und wenigstens einen Wärmeübertrager auf, wobei die Speichereinrichtung ausgebildet ist zur Speicherung eines sauerstoffhaltigen Fluides in dem Fluidspeicher und zur Bereitstellung eines gasförmigen oder flüssigen Entnahmestroms des sauerstoffhaltigen Fluides, wobei der Wärmeübertrager ausgebildet ist zur Kühlung des bereitgestellten Entnahmestroms durch Wärmeübertragung auf einen Kühlstrom eines gespeicherten und ebenfalls von der Speichereinrichtung bereitgestellten Fluides und wobei aus dem Wärmeübertrager ein gekühlter, sauerstoffhaltiger Gasstrom bzw. Atemstrom zur Zuführung zum Körper austritt.

Ein Verfahren kann dementsprechend vorsehen, dass in dem Fluidspeicher ein sauerstoffhaltiges Fluid gespeichert und ein Entnahmestrom des sauerstoffhaltigen Fluides bereitgestellt wird, wobei der Entnahmestrom mit einem Kühlstrom gekühlt wird, um einen gekühlten, sauerstoffhaltigen Gasstrom mit vorzugsweise definierter Temperatur zur Zuführung zum Körper zu erhalten. Zur Bereitstellung eines gekühlten sauerstoffhaltigen Gases mit einer bestimmten Temperatur unterhalb der Körpertemperatur kann ein kaltes, vorzugsweise verflüssigtes, Gas in einem ersten Teilstrom erwärmt und anschließend mit einem zweiten, kalten Bypass-Teilstrom vermischt werden, wodurch die Temperatur des Gases in einfacher Weise gesteuert und/oder geregelt werden kann.

Es kann eine Speicherung des sauerstoffhaltigen Fluides in flüssiger Form vorgesehen sein, wobei ein Entnahmestrom des sauerstoffhaltigen Fluides von der Speichereinrichtung tiefkalt in flüssigem oder gasförmigen Zustand bereitgestellt wird und vor der Zuführung zu einem Patienten zunächst auf eine bestimmte Temperatur erwärmt wird. Hierzu kann ein Vorwärmer bzw. ein atmosphärischer Verdampfer vorgesehen sein, aus dem der erwärmte Entnahmestrom austritt. Um den Entnahmestrom danach auf eine bestimmte, therapeutisch wirksame tiefere Temperatur abzukühlen, sieht die Erfindung die Kühlung des erwärmten Entnahmestroms mit einem Kühlstrom eines gespeicherten und von der Speichereinrichtung ebenfalls bereitgestellten Fluides vor. Entsprechendes gilt für den Fall, dass das sauerstoffhaltige Gas unter hohem Druck gespeichert wird, wobei es bei adiabater Entspannung des Gases beim Austritt aus dem Fluidspeicher zu einer deutlichen Temperaturabsenkung kommt. Um hier eine bestimmte, tiefe Temperatur des Gases für die Zuführung zu einem Patienten zu gewährleisten, kann es erforderlich sein, das kalte Gas nach der Entspannung zunächst zu erwärmen und anschließend dann wieder gezielt auf eine bestimmte Temperatur unterhalb der Körpertemperatur zu kühlen.

Der Erfindung liegt der Grundgedanke zugrunde, einen von der Speichereinrichtung bereitgestellten Entnahmestrom eines sauerstoffhaltigen Fluides mittels eines zweiten, kälteren und ebenfalls von der Speichereinrichtung bereitgestellten Kühlstroms durch direkte und/oder indirekte Wärmeübertragung auf den Kühlstrom abzukühlen, so dass ein gekühlter sauerstoffhaltiger Gasstrom mit einer bestimmten Temperatur als Atemstrom zur Zuführung zum Körper eines Patienten erhalten wird. Insbesondere ermöglicht es die Erfindung damit, eine vorteilhafte Temperatur des Gasstroms für die Zuführung zu einem Körper in einfacher Weise einzustellen bzw. die Temperatur des Gasstroms zu steuern und/oder zu regeln, so dass Langzeitfolgen eines Schlaganfalls durch die Gaszuführung verhindert oder zumindest abgeschwächt werden können. Von Vorteil für den Behandlungserfolg ist auch der Umstand, dass mit der erfindungsgemäß vorgesehenen Kühlung des Entnahmestroms des sauerstoffhaltigen Fluides mit dem zweiten, kälteren Kühlstrom die Temperatur des Entnahmestroms näherungsweise konstant auf einen vorgegebenen Temperaturniveau gehalten werden kann. Die erfindungsgemäße Vorrichtung ist neben der Behandlung eines Patienten bei einem Schlaganfall auch zur Behandlung und Abminderungen der Langzeitfolgen bei Patienten mit einem Schädelhirntrauma, mit einer Gehirnerschütterung oder einem Hitzeschlag geeignet. Des Weiteren wirkt sich ein Herabsenken der Körpertemperatur auch positiv auf die Überlebenschancen bei einer Reanimation aus, da der Sauerstoffbedarf des Patienten verringert wird.

Mit der erfindungsgemäßen Vorrichtung lässt sich in einfacher und kostengünstiger Weise ein gekühlter, sauerstoffhaltiger Gasstrom zur Zuführung zum Körper bei einer definierten, für die Therapie geeigneten, tiefen Temperatur bereitstellen, wobei Temperaturabweichungen aufgrund von Umgebungseinflüssen, wie beispielsweise aufgrund hoher Umgebungstemperaturen im Sommer, durch Kühlung des Entnahmestroms aus dem Fluidspeicher mit einem kälteren Fluid verringert bzw. ausgeschlossen werden können. Auf diese Weise wird eine sichere Versorgung des Patienten mit einem gleichbleibend gekühlten sauerstoffhaltigen Gasstrom erreicht. Die Temperatur des gekühlten sauerstoffhaltigen Gasstroms liegt selbstverständlich unterhalb der Körpertemperatur des Patienten, um eine therapeutische Wirkung zu erzielen. Vorzugsweise beträgt die Temperatur des dem Patienten zugeführten Gasstroms weniger als 5 °C, weiter bevorzugt weniger als 0 °C, vorteilhafterweise zwischen -10 °C und -32 °C.

Erleidet ein Patient einen Schlaganfall, können Rettungskräfte, wie beispielsweise Rettungssanitäter oder Notärzte, die erfindungsgemäße transportable Vorrichtung auf einfache Weise mit sich führen. Die Vorrichtung kann dabei beispielsweise in einem Rettungswagen, Notarztwagen oder Rettungshubschrauber über weite Strecken transportiert werden. Darüber hinaus eignet sich die Vorrichtung auch dazu, auf einfache Art und Weise von einer einzelnen Person getragen zu werden. Auf diese Weise ist eine schnelle und frühzeitige Behandlung und Therapie des Patienten auch außerhalb eines Krankenhauses möglich.

Die Speichereinrichtung der erfindungsgemäßen Vorrichtung kann vorzugsweise derart dimensioniert sein bzw. das Fassungsvermögen des wenigstens einen Fluidspeichers kann so bemessen sein, dass die Bereitstellung des Entnahmestroms und/oder des Kühlstroms lediglich über einen Zeitraum von wenigen Stunden, vorzugsweise von weniger als zwei Stunden, weiter vorzugsweise von weniger als einer Stunde, sichergestellt ist. Die erfindungsgemäße Vorrichtung ist dementsprechend insbesondere für eine Erstversorgung eines Patienten vorgesehen. Ein geringes Speichervermögen der Speichereinrichtung führt zu einer Gewichtseinsparung bei der erfindungsgemäßen Vorrichtung und zu einer Verringerung des Bauvolumens, was den Transport der Vorrichtung erleichtert. Zudem können so Herstellungskosten verringert werden. Gleichzeitig muss die Speichereinrichtung hinreichend groß ausgebildet sein, damit im Notfall ein Patient ausreichend lang mit einem gekühlten sauerstoffhaltigen Gasstrom versorgt werden kann. Mit hinreichend lang ist der bereits angesprochene Zeitraum von wenigen Stunden gemeint. Die transportable Vorrichtung wird vorzugsweise in Notfällen eingesetzt, wobei die Versorgung des Patienten mit einem gekühlten sauerstoffhaltigen Gasstrom vorzugsweise zumindest bis zum Eintreffen des Patienten in einem Krankenhaus sichergestellt sein soll.

Der Fluidspeicher ist vorzugsweise als wiederbefüllbarer Speicher ausgebildet. Die transportable Vorrichtung lässt sich dann durch Wiederauffüllen des Fluidspeichers mehrfach verwenden. Alternativ ist ein Austausch des Fluidspeichers möglich.

Vorzugsweise ist eine Atemmaske zum Zuführen des gekühlten sauerstoffhaltigen Gasstroms in die Atemwege des Patienten vorgesehen. Entsprechende Atemmasken sind dem Fachmann bekannt. Die Atemmasken können dabei Teile des Gesichts abdecken oder das gesamte Gesicht bedecken. Eine Maske kann dabei eine Zuführung des Gases über den Mund und/oder die Nase des Patienten vorsehen. Zu diesem Zweck kann die Maske wenigstens einen Tubus aufweisen. Durch die Atemmaske wird eine sichere Zuführung des gekühlten sauerstoffhaltigen Gasstroms in die Atemwege des Patienten sichergestellt.

Bei einer bevorzugten Ausführungsform der Erfindung ist eine Steuer- und/oder Regelungseinrichtung vorgesehen. Diese ist ausgebildet zur Steuerung und/oder Regelung der Temperatur des für die Zuführung zum Patienten vorgesehenen gekühlten sauerstoffhaltigen Gasstroms. Die Steuerung und/oder Regelung der Temperatur erfolgt durch Veränderung des Massenverhältnisses von Entnahmestrom und Kühlstrom, mit dem der Entnahmestrom aus dem Fluidspeicher gekühlt wird. Mithilfe der Steuer- und/oder Regelungseinrichtung kann die Temperatur des gekühlten sauerstoffhaltigen Gasstroms bzw. des dem Patienten zugeführten Atemstroms auf einen gewünschten Sollwert eingestellt werden. Gleichzeitig kann die Temperatur des Gasstroms auf einfache Weise konstant gehalten werden, auch bei sich stark und schnell ändernden Umgebungstemperaturen. Darüber hinaus ist es auch möglich, einen Temperaturverlauf des gekühlten sauerstoffhaltigen Gasstroms einzustellen, was sich positiv auf den Abkühlvorgang des Gewebes eines Patienten auswirken kann. So kann beispielsweise die Körpertemperatur eines Patienten innerhalb eines kurzen Zeitraums herabgesenkt werden, in dem eine geringe Temperatur des gekühlten sauerstoffhaltigen Gasstroms eingestellt wird. Ist die gewünschte Körpertemperatur des Patienten erreicht, kann zur Aufrechterhaltung der herabgesenkten Körpertemperatur eine höhere Temperatur des gekühlten sauerstoffhaltigen Gasstroms eingestellt werden.

Die Steuer- und/oder Regelungseinrichtung kann ein aktiv und/oder passiv angesteuertes Ventil aufweisen. Als aktives Ventil kann beispielsweise ein elektrisch schaltbares Ventil vorgesehen sein. Werden schaltbare Ventile, also eine Steuer- und/oder Regelungseinrichtung mit wenigstens einem aktiven Aktuator, eingesetzt, ist es notwendig, die Temperatur des gekühlten sauerstoffhaltigen Gasstroms zur Zuführung zum Körper des Patienten zu messen. Abhängig von den gemessenen Temperaturen wird dann das Ventil aktiv geschaltet. Der Einsatz von aktiv geschalteten Ventilen weist den Nachteil auf, dass Energie zum Messen der Temperatur, zum Auswerten der Temperatur und zum Schalten der Ventile benötigt und verbraucht wird. Die benötigte Energie kann beispielsweise einem elektrischen Speicher, einem Seebeck-Element und/oder einem in der Speichereinrichtung gespeicherten Fluid entnommen werden.

Vorzugsweise weist die Steuer- und Regelungseinrichtung aber ein passives Ventil auf, das über einen Bimetall-Aktuator geschaltet wird. Ein Bimetall, auch Thermobimetall genannt, besteht aus zwei Schichten unterschiedlicher Metalle, die miteinander stoffschlüssig oder formschlüssig verbunden sind. Eine Temperaturänderung bewirkt eine Veränderung der Form des Bimetalls. Diese äußert sich beispielsweise als Verbiegung, die durch unterschiedliche Wärmeausdehnungskoeffizienten der verwendeten Metalle bewirkt wird. Weicht die Temperatur des gekühlten sauerstoffhaltigen Gasstroms vom Sollwert ab, ändert sich die Form des für den Anwendungsfall angepassten Bimetalls, wodurch das Ventil geschaltet bzw. verstellt und die Größe des Kühlstroms verändert wird. Durch den Einsatz eines Bimetalls als Aktuator einer Steuer- und/oder Regelungseinrichtung lässt sich eine Steuerung und/oder Regelung der Temperatur des gekühlten sauerstoffhaltigen Gasstroms auf einfache Weise erreichen. Der Einsatz von aktiv geschalteten Aktuatoren ist dann nicht notwendig, wodurch der Energieverbrauch bzw. der Energiebedarf und die Komplexität der Steuer- und/oder Regelung deutlich reduziert werden können.

Bei der Erfindung wird der Kühlstrom durch einen Bypassstrom des in dem Fluidbehälter gespeicherten sauerstoffhaltigen Fluides gebildet. Der Entnahmestrom und der Kühlstrom weisen eine gleiche Zusammensetzung auf, wobei ein erwärmter erster Massenstrom des gespeicherten sauerstoffhaltigen Fluides mit einem zweiten Massenstrom desselben sauerstoffhaltigen Fluides (Bypassstrom) auf eine bestimmte, tiefere Temperatur gekühlt wird. Erfindungsgemäß werden dem Fluidbehälter dann zwei Massenströme entnommen, wobei der eine Massenstrom zur Temperatursteuerung und/oder -regelung des anderen Massenstroms im Sinne einer Bypass-Lösung verwendet wird. Erfolgt die Kühlung durch direkte Wärmeübertragung zwischen dem Entnahmestrom und dem Kühlstrom, wobei es zum Stoffübergang bzw. zur Vermischung der beiden Massenströme kommt, lässt sich auch bei unterschiedlicher Größe der beiden Massenströme stets eine gleiche Zusammensetzung des dem Patienten zugeführten gekühlten Atemgases erreichen. Gleichzeitig kann die Temperatursteuerung- und/oder regelung vereinfacht werden, da beide Massenströme die gleichen physikalischen Eigenschaften aufweisen. Grundsätzlich ist aber auch nicht ausgeschlossen, den Entnahmestrom mit einem Fluid mit anderer Zusammensetzung zu kühlen. Dies schafft die Möglichkeit, beispielsweise eine therapeutisch wirksame Gaskomponente, wie ein Edelgas, zuzuführen. Die Kühlung des Entnahmestroms mit einem Bypassstrom, wobei beide Massenströme aus demselben Fluidbehälter entnommen werden, führt zu einem vereinfachten konstruktiven Aufbau und lässt eine kompakte Bauweise der erfindungsgemäßen Vorrichtung zu. Insbesondere entfällt die Notwendigkeit, eine separate Kühleinrichtung zur Bereitstellung eines Kühlstroms zur Abkühlung des entnommenen Massenstroms des sauerstoffhaltigen Gases bereitzustellen.

In einer vorteilhaften Ausführungsform der Erfindung enthält der Fluidspeicher ein tiefgekühltes, verflüssigtes sauerstoffhaltiges Fluid, insbesondere verflüssigten Sauerstoff oder verflüssigte Luft. Dadurch werden die Anforderungen an die Speichertechnologie im Vergleich zu einer Druckgasspeicherung verringert, wobei auf hohe Systemdrücke verzichtet werden kann.

Vorteilhafterweise ist der Fluidspeicher derart isoliert, dass ein tiefgekühltes, verflüssigtes sauerstoffhaltiges Fluid über einen ausreichend langen Zeitraum eine ausreichend tiefe Temperatur aufweist. Als ausreichender Zeitraum sind wenigstens zwei Tage, vorzugsweise wenigstens drei Tage, weiter vorzugsweise wenigstens vier Tage, vorteilhaft sieben Tage, anzusehen.

Trotz Isolierung des Fluidspeichers lässt sich jedoch bei einer Tieftemperaturspeicherung des sauerstoffreichen Fluides eine Wärmeübertragung von der Umgebung in den Fluidspeicher nicht völlig ausschließen, wobei der Behälterinnendruck aufgrund der Wärmeübertragung zunimmt und eine Fluidentnahme und die Zufuhr des sauerstoffreichen Gases zum Patienten allein aufgrund des Behälterinnendrucks ohne zusätzliche druckaufbauende Einrichtungen, wie Gebläse oder dergleichen, erfolgen kann.

Grundsätzlich ist aber auch eine Druckspeicherung nicht ausgeschlossen, vorzugsweise mit anschließender adiabater Entspannung des gespeicherten sauerstoffreichen Fluides. Der Fluidspeicher kann hierbei ein gasförmiges sauerstoffhaltiges Fluid unter hohem Druck enthalten. Der Druck kann dabei vorzugsweise wenigstens 20 bar aufweisen, bevorzugt wenigstens 50 bar, weiter bevorzugt zwischen 100 bar und 400 bar oder mehr, sogar bis 800 bar. Auf diese Weise lässt sich eine hohe Menge eines gasförmigen Fluides in einem kleinen Volumen speichern und vorhalten. Bei einer vorzugsweisen adiabaten Entspannung des Gases kann die Temperatur des Gases beim Austreten aus dem Fluidspeicher stark abgesenkt werden. Auf diese Weise lässt sich aus dem Fluidspeicher ein Bypassstrom mit ausreichend geringer Temperatur bereitstellen. Darüber hinaus kann der Behälterinnendruck ausreichen, um alle Systemdruckverluste zu kompensieren und ohne zusätzliche druckaufbauende Einrichtungen einen gekühlten sauerstoffhaltigen Gasstrom aus dem Fluidspeicher den Atemwegen eines Patienten zuzuführen.

Bei einer weiter bevorzugten Ausführungsform der Erfindung weist der Entnahmestrom und/oder der Kühlstrom einen Sauerstoffgehalt von wenigstens 20 Gew.% auf, wobei es sich bei beiden Strömen um tiefkalte, vorzugsweise verflüssigte, Luft handeln kann. Der Sauerstoffgehalt kann aber auch mehr als 50 Gew.-%, weiter vorzugsweise mehr als 90 Gew.-%, betragen. Es kann auch reiner Sauerstoff eingesetzt werden.

Besonders bevorzugt weist der Entnahmestrom und/oder der Kühlstrom keine additiven Gaskomponenten mit therapeutischer Wirkung auf. Der Entnahmestrom und/oder der Kühlstrom können dann als Komponenten - mit Ausnahme von Spurenbestandteilen - lediglich Sauerstoff und gegebenenfalls Sichtstoff aufweisen. Ein hoher Sauerstoffanteil wirkt sich positiv auf die Behandlung des Patienten aus, da auf einfache Weise eine Sauerstoffsättigung des Gewebes erzielt werden kann. Allerdings ist auch die Verwendung von sauerstoffreichen Gasen für die Patientenversorgung möglich, die wenigstens eine zusätzliche, therapeutisch wirksame Komponente aufweisen, beispielsweise ein Edelgas, wie Argon und/oder Helium. Als zusätzliche Komponente können aber auch Vasodilatatoren eingesetzt werden, wie beispielsweise Stickstoffmonoxid. Als Vasodilatatoren werden gefäßerweiternde und damit blutdrucksenkende Substanzen bezeichnet. Durch die Zugabe von Vasodilatatoren kann ein positiver Einfluss auf die Durchblutung des Gewebes und damit auf die Wärmeübertragung und auf die Temperatur des Gewebes des Patienten erzielt werden. Insbesondere durch den kombinierten Einsatz wenigstens eines Inertgases mit wenigstens einem Vasodilatator als zusätzliche Komponente des sauerstoffhaltigen Gasstroms lässt sich eine schnelle und verbesserte Absenkung der Körpertemperatur erreichen.

Bei einer bevorzugten Ausführungsform werden der Entnahmestrom des sauerstoffhaltigen Fluides und der Kühlstrom von der Speichereinrichtung auf einem im Wesentlichen gleichen Temperaturniveau bereitgestellt. Der Entnahmestrom und der Kühlstrom können einer gemeinsamen Leitung, die mit dem Fluidspeicher der Speichereinrichtung verbunden ist, entnommen werden. Mittels eines einfachen Y-Rohrs oder mittels eines Dreiwegeventils können zwei getrennte Massenströme des sauerstoffhaltigen Fluides bereitgestellt werden, wovon ein Massenstrom einen Bypassstrom zur Kühlung des anderen Massenstroms bildet. So kann ein einfacher und kostengünstiger Aufbau der Vorrichtung erreicht werden.

Bei einer weiter bevorzugten Ausführungsform der Erfindung ist wenigstens ein Vorwärmer zur Vorwärmung des Entnahmestroms des sauerstoffhaltigen Fluides vorgesehen, der dem Wärmeübertrager in Strömungsrichtung des Entnahmestroms vorgeordnet ist. Der Entnahmestrom wird in dem Vorwärmer von einer Austrittstemperatur beim Austritt aus der Speichereinrichtung auf eine Temperatur oberhalb der Temperatur des Kühlstroms erwärmt, insbesondere durch Wärmeübertragung aus der Umgebung(sluft). Der Vorwärmer kann als atmosphärischer Verdampfer ausgebildet sein, der die Energie der umgebenden Atmosphäre, also der Umgebungsluft, nutzt. Das kann insbesondere dann vorgesehen sein, wenn ein verflüssigtes sauerstoffhaltiges Gas in dem Fluidspeicher gespeichert wird. Entsprechende Vorwärmer und/oder Verdampfer sind dem Fachmann bekannt. Aus dem Vorwärmer tritt dann ein erwärmter sauerstoffhaltiger Gasstrom aus, der dann anschließend mit einem gasförmigen oder ggf. auch flüssigen Bypassstrom des kalten sauerstoffhaltigen Gases aus dem Fluidspeicher gekühlt wird, um eine bestimmte tiefe Temperatur für die Zuführung des sauerstoffhaltigen Gasstroms in die Atemwege des Patienten zu erreichen. Mithilfe des Vorwärmers kann auf einfache Weise sichergestellt werden, dass ein ausreichender Temperaturunterschied zwischen dem Entnahmestrom und dem Kühlstrom erreicht wird, was das exakte Einstellen einer gewünschten Temperatur des sauerstoffhaltigen Gasstromes ermöglicht. Die Temperatur des Gasstromes, der dem Patienten zugeführt wird, kann dann auf einfache Art mithilfe des zweiten, kälteren Bypassstroms eingestellt werden.

Erfindungsgemäß wird der gekühlte, sauerstoffhaltige Gasstrom durch eine zumindest teilweise Zusammenführung bzw. Vermischung des Entnahmestroms und des Kühlstroms erhalten. Dabei können die beiden Massenströme auch vollständig miteinander vermischt werden. Auf diese Weise wird eine direkte Wärmeübertragung zwischen dem ersten und dem zweiten Massenstrom erreicht, was zu einer schnellen und effektiven Kühlung des Entnahmestroms führt. Eine vollständige Zusammenführung des Entnahmestroms und des Kühlstroms weist darüber hinaus den Vorteil auf, dass Gasverluste verhindert werden können. Die eingesetzten Fluid-Ressourcen werden so optimal genutzt.

In einer weiter vorteilhaften Ausführungsform der Erfindung kann zwischen dem Vorwärmer und dem Wärmeübertrager wenigstens ein weiterer Wärmeübertrager zur Vorkühlung des Entnahmestroms durch indirekte Wärmeübertragung auf den Kühlstrom vorgesehen sein. Falls der Kühlstrom in flüssigem Zustand in den weiteren Wärmeübertrager eintritt, kann der Kühlstrom aufgrund der Wärmeübertragung von dem Entnahmestrom im weiteren Wärmeübertrager zumindest teilweise verdampfen.

Das erfindungsgemäße System weist wenigstens einen stationär angeordneten Vorratsspeicher zur Befüllung des Fluidspeichers der erfindungsgemäßen Vorrichtung auf, wobei das System zur Herstellung einer lösbaren Fluidverbindung zwischen dem Fluidspeicher und dem Vorratsspeicher und zur Wiederbefüllung des Fluidspeichers der Vorrichtung aus dem Vorratsspeicher ausgebildet ist.

Der stationär angeordnete Vorratsspeicher, auch Base Unit genannt, bevorratet eine große Menge des sauerstoffhaltigen Fluides, vorzugsweise unter einem hohen Druck, wobei das Fluid vorzugsweise wenigstens anteilig als flüssige Phase vorliegt. Das Fluid kann dabei unter einem sehr hohen Druck von mehreren 100 bar gespeichert sein, bevorzugt von mehr als 400 bar. Zum Wiederbefüllen des Fluidspeichers der erfindungsgemäßen Vorrichtung wird der Fluidspeicher mit dem stationär angeordneten Vorratsspeicher verbunden. Die Verbindung kann dabei beispielsweise über eine Bajonett-, eine Schraub- oder Steckverbindung auf sichere Weise erzielt werden. Die Wiederbefüllung des Fluidspeichers der transportablen Vorrichtung erfolgt dabei vorzugsweise innerhalb weniger Minuten, bevorzugt in weniger als 10 Minuten, weiter bevorzugt in weniger als fünf Minuten, vorteilhaft in ungefähr einer Minute. Der stationär angeordnete Vorratsspeicher ist derart ausgebildet, dass das gespeicherte Fluid über einen langen Zeitraum bevorratet werden kann. Dabei ist das Füllvolumen des stationär angeordneten Vorratsspeichers ausreichend groß, um den Fluidspeicher der transportablen Vorrichtung mehrfach vollständig zu beladen. Es ist vorteilhaft, wenn der stationär angeordnete Vorratsspeicher eine ausreichende Menge des sauerstoffhaltigen Fluides speichert, um den Fluidspeicher der transportablen Vorrichtung wenigstens zweimal vollständig wieder aufzufüllen, weiter bevorzugt wenigstens fünfmal, besonders vorteilhaft wenigstens zehnmal wieder aufzufüllen.

Weitere Merkmale der vorliegenden Erfindung ergeben sich aus der nachfolgenden Beschreibung eines bevorzugten Ausführungsbeispiels der Erfindung anhand der Zeichnung und aus der Zeichnung selbst.

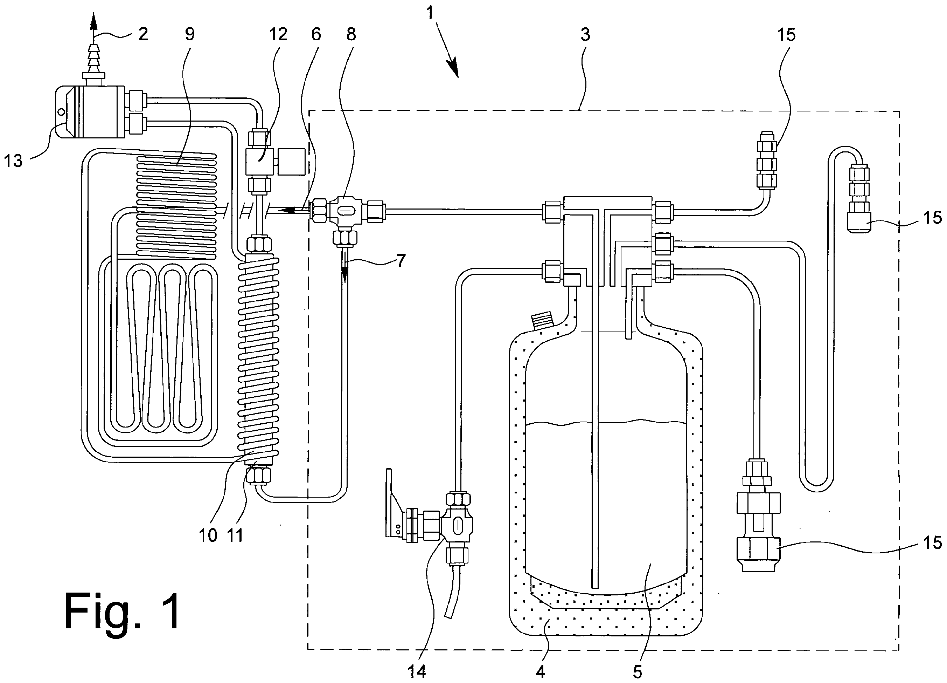

Die Erfindung wird im Folgenden anhand von Figur 1 erläutert. Sie zeigt eine schematische Darstellung einer transportablen Vorrichtung zur Bereitstellung eines gekühlten, sauerstoffhaltigen Gasstroms für die Zuführung zu einem Körper eines Säugetiers über die Atemwege, insbesondere eines Menschen, um die Körpertemperatur abzusenken.

Fig. 1 zeigt eine transportable Vorrichtung 1 zur Bereitstellung eines gekühlten sauerstoffhaltigen Gasstroms 2 für die Zuführung zu einem nicht dargestellten Körper eines Säugetieres über die Atemwege, insbesondere eines Menschen, um die Körpertemperatur abzusenken. Die transportable Vorrichtung 1 weist eine Speichereinrichtung 3 mit wenigstens einem Fluidspeicher 4 auf. Der Fluidspeicher 4 bevorratet wenigstens ein sauerstoffhaltiges Fluid 5, das vorzugsweise als kaltverflüssigtes Gas in dem Fluidspeicher 4 der Speichereinrichtung 3 gespeichert ist. Anders als in Fig. 1 dargestellt, kann die Speichereinrichtung 3 auch mehr als einen Fluidspeicher 4 aufweisen, wobei die unterschiedlichen Fluidspeicher 4 verschiedene aber auch gleiche Fluide 5 bevorraten können.

Die Speichereinrichtung 3 stellt einen Entnahmestrom 6 eines sauerstoffhaltigen Fluides 5 und einen Kühlstrom 7 des Fluides 5 bereit. Die beiden Massenströme 6, 7 werden dem Fluidspeicher 4 über eine gemeinsame Rohrleitung und ein gemeinsames Ventil entnommen. Ein Dreiwegeventil 8 der Speichereinrichtung 3 teilt den gemeinsamen Massenstrom in den ersten Massenstrom 6 und den zweiten Massenstrom 7 auf. Das Ventil 8 kann auch als Y-Rohr ausgebildet sein. Das Ventil 8 kann dabei schalt- und/oder regelbar ausgebildet sein, wodurch die Massenströme 6, 7 verändert werden können.

Der Entnahmestrom 6 wird durch einen Vorwärmer 9 geleitet. Handelt es sich bei dem Fluid 5 um ein kaltes, verflüssigtes Gas, kann der Vorwärmer 9 als atmosphärischer Verdampfer ausgebildet sein. Die zur Verdampfung des verflüssigten Gases benötigte Energie wird dabei der Umgebungsluft entnommen. Bei der Verdampfung steigt der Druck, so dass Systemdruckverluste überwunden werden und die Zuführung des kalten sauerstoffhaltigen Gasstroms 2 in die Atemwege eines Patienten auch ohne zusätzliche Fördereinrichtungen, wie Verdichter oder Gebläse, sichergestellt ist. Beim Austritt aus dem Vorwärmer 9 weist der Entnahmestrom 6 dann eine Temperatur auf, die oberhalb der Temperatur des Kühlstroms 7 liegt.

Aufgrund eines Wärmeübergangs von der Umgebung in den Fluidbehälter 4 kann auch der Behälterinnendruck ansteigen, so dass allein der Behälterinnendruck ausreichen kann, um Systemdruckverluste zu überwinden und die Zuführung des kalten sauerstoffhaltigen Gasstroms 2 in die Atemwege eines Patienten sichergestellt wird.

Dem Vorwärmer 9 kann ein indirekter Wärmeübertrager 10 nachgeschaltet sein, wobei Wärme vom Entnahmestrom 6 auf den Kühlstrom 7 übertragen und der Entnahmestrom 6 abgekühlt wird. Zur Verbesserung der Wärmeübertragung in dem Wärmeübertrager 10 kann eine metallische Masse 11 mit einer hohen Wärmekapazität vorgesehen sein. Durch die metallische Masse kann die Wärmeübertragung verbessert und auf einem konstanten Maß gehalten werden. Als Material der metallischen Masse kann beispielsweise Kupfer oder Messing eingesetzt werden. Es sind aber auch Ausführungsformen der erfindungsgemäßen Vorrichtung 1 möglich, bei denen kein indirekter Wärmeübertrager 10 vorgesehen ist.

Die Größe des Kühlstroms 7 wird von einem Aktuator einer Steuer- und/oder Regelungseinrichtung 12 gesteuert und/oder geregelt. Der Aktuator kann als aktiv geschaltetes Ventil ausgebildet sein. Zur Schaltung des Ventils wird die Ist-Temperatur des sauerstoffhaltigen Gasstroms 2 gemessen, mit einem Sollwert verglichen und der aktive Aktuator 12 dementsprechend geschaltet. In einer vorteilhaften Ausführungsform handelt es sich bei dem Aktuator 12 um einen passiven Aktuator. Als passiver Aktuator 12 kann ein Bimetall eingesetzt werden, das bei Veränderung der das Bimetall umgebenden Temperatur seine Form verändert, wodurch die Größe des Kühlstroms 7 mittels des Aktuators 12 verändert werden kann. Durch Veränderung der Massenverhältnisse von Entnahmestrom 6 und Kühlstrom 7 lässt sich die Abkühlung des Entnahmestroms 6 in dem Wärmeübertrager 10 beeinflussen.

Der Entnahmestrom 6 wird anschließend einem direkten Wärmeübertrager 13 zugeführt. In dem Wärmeübertrager 13 werden der Entnahmestrom 6 und der Kühlstrom 7 vorzugsweise vollständig oder auch nur teilweise zusammengeführt. Nicht zusammengeführte Anteile der Massenströme 6 und 7 können an die Umgebung abgegeben werden. Durch das Vermischen des Entnahmestroms 6 mit dem Kühlstrom 7 wird die Temperatur des Entnahmestroms 6 vorzugsweise weiter herabgesetzt, so dass ein gekühlter, sauerstoffhaltiger Gasstrom 2 bei einer bestimmten, therapeutisch wirksamen Temperatur unterhalb der Körpertemperatur bereitgestellt wird. Grundsätzlich kann es auch bereits in dem ersten Wärmeübertrager 10 zu einer Temperaturangleichung des Entnahmestroms 6 und des Kühlstroms 7 kommen, so dass im Wesentlichen gleich temperierte Massenströme 6, 7 in dem direkten Wärmeübertrager 13 zusammengeführt werden.

In besonders vorteilhafter Weise werden der Entnahmestrom 6 und der Kühlstrom 7 vollständig in dem direkten Wärmeübertrager 13 miteinander vermischt, so dass kein Fluid 5 an die Umgebung abgegeben wird.

Der in Fig. 1 dargestellte Fluidspeicher 4 ist wiederbefüllbar. Dazu weist der Fluidspeicher 4 eine Schlauch- und/oder Rohrverbindung mit einem Zuflussventil 14 auf. Das Zuflussventil 14 kann dabei mit einem nicht dargestellten stationär angeordneten Vorratsspeicher verbunden werden. Über das Zuflussventil 14 kann der Fluidspeicher 4 auf einfache Weise wiederbefüllt werden.

Des Weiteren weist die transportable Vorrichtung 1 an sich bekannte Sicherheitseinrichtungen für eine sichere Lagerung und einen sicheren Betrieb der Vorrichtung auf, wie beispielhaft dargestellte Sicherheitsventile 15.

### Bezugszeichenliste:

- 1: Vorrichtung
- 2: Gasstrom
- 3: Speichereinrichtung
- 4: Fluidspeicher
- 5: Fluid
- 6: Massenstrom
- 7: Massenstrom
- 8: Ventil
- 9: Vorwärmer
- 10: Wärmeübertrager
- 11: Masse
- 12: Regelungseinrichtung
- 13: Wärmeübertrager
- 14: Zuflussventil
- 15: Sicherheitsventil

## Patentansprüche

1. Transportable Vorrichtung (1) zur Bereitstellung eines gekühlten sauerstoffhaltigen Gasstroms (2) für die Zuführung zu einem Körper eines Säugetiers über die Atemwege, insbesondere eines Menschen, um die Körpertemperatur abzusenken,
mit einer wenigstens einen Fluidspeicher (4) aufweisenden Speichereinrichtung (3) und mit wenigstens einem Wärmeübertrager (10, 13),
wobei die Speichereinrichtung (3) ausgebildet ist zur Speicherung eines sauerstoffhaltigen Fluides (5) in dem Fluidspeicher (4) und zur Bereitstellung eines Entnahmestroms (6) des sauerstoffhaltigen Fluides (5) aus dem Fluidspeicher (4),
wobei der Wärmeübertrager (10, 13) ausgebildet ist zur Kühlung des aus dem Fluidspeicher (4) bereitgestellten Entnahmestroms (6) durch Wärmeübertragung auf einen Kühlstrom (7) eines gespeicherten und von der Speichereinrichtung (3) bereitgestellten Fluides (5) vor der Zuführung des Entnahmestroms (6) zum Körper,
wobei aus dem Wärmeübertrager (10, 13) ein gekühlter, sauerstoffhaltiger Gasstrom (2) zur Zuführung zum Körper austritt und
wobei der Entnahmestrom (6) nach seinem Austritt aus dem Fluidspeicher (4) und vor seiner Zuführung zum Körper durch den Kühlstrom (7) gekühlt wird,
**dadurch gekennzeichnet,**
**dass** der Kühlstrom (7) durch einen Bypassstrom des in dem Fluidbehälter (4) gespeicherten sauerstoffhaltigen Fluides (5) gebildet wird, wobei der Entnahmestrom (6) und der Kühlstrom (7) eine gleiche Zusammensetzung aufweisen und wobei ein erwärmter erster Massenstrom des gespeicherten sauerstoffhaltigen Fluides (5) mit einem zweiten Massenstrom desselben sauerstoffhaltigen Fluides (5) als Bypassstrom auf eine bestimmte, tiefere Temperatur gekühlt wird und wobei der gekühlte, sauerstoffhaltige Gasstrom (2) durch eine wenigstens teilweise Zusammenführung des Entnahmestroms (6) und des Kühlstroms (7) erhältlich ist.

2. Transportable Vorrichtung (1) nach Anspruch 1,
**dadurch gekennzeichnet,**
**dass** der Wärmeübertrager (13) zur direkten Wärmeübertragung zwischen dem Entnahmestrom (6) und dem Kühlstrom (7) durch Vermischung von Entnahmestrom (6) und Kühlstrom (7) ausgebildet ist.

3. Transportable Vorrichtung (1) nach Anspruch 1 oder 2, **dadurch gekennzeichnet, dass** eine Steuer- und/oder Regelungseinrichtung (12) ausgebildet zur Steuerung und/oder Regelung der Temperatur des gekühlten sauerstoffhaltigen Gasstroms (2) durch Veränderung der Größe des Kühlstroms (7) vorgesehen ist.

4. Transportable Vorrichtung (1) nach einem der vorhergehenden Ansprüche, **dadurch gekennzeichnet, dass** der Fluidspeicher (4) ein tiefgekühltes, verflüssigtes sauerstoffhaltiges Fluid (5), insbesondere flüssigen Sauerstoff oder verflüssigte Luft, enthält.

5. Transportable Vorrichtung (1) nach einem der vorhergehenden Ansprüche, **dadurch gekennzeichnet, dass** der Entnahmestrom (6) und/oder der Kühlstrom (7) einen Sauerstoffgehalt von wenigstens 20 Gew.-%, vorzugsweise von mehr als 50 Gew.-%, weiter vorzugsweise von mehr als 90 Gew.-%, aufweist.

6. Transportable Vorrichtung (1) nach einem der vorhergehenden Ansprüche, **dadurch gekennzeichnet, dass** der Entnahmestrom (6) und der Kühlstrom (7) von der Speichereinrichtung (3) auf einem gleichen Temperaturniveau bereitgestellt werden.

7. Transportable Vorrichtung (1) nach einem der vorhergehenden Ansprüche, **dadurch gekennzeichnet, dass** wenigstens ein dem Wärmeübertrager (10, 13) in Strömungsrichtung des Entnahmestroms (6) vorgeordneter Vorwärmer (9) zur Vorwärmung des Entnahmestroms (6) vorgesehen ist, wobei der Entnahmestrom (6) in dem Vorwärmer von einer Austrittstemperatur beim Austritt aus der Speichereinrichtung (3) auf eine Temperatur oberhalb der Temperatur des Kühlstroms (7) erwärmt wird, insbesondere durch Wärmeübertragung aus der Umgebung.

8. Transportable Vorrichtung (1) nach Anspruch 7, **dadurch gekennzeichnet, dass** zwischen dem Vorwärmer (9) und dem Wärmeübertrager (13) wenigstens ein weiterer Wärmeübertrager (10) zur Vorkühlung des Entnahmestroms (6) durch indirekte Wärmeübertragung auf den Kühlstrom (7) vorgesehen ist.

9. System mit wenigstens einer Vorrichtung (1) nach einem der vorhergehenden Ansprüche und mit einem stationär angeordneten Vorratsspeicher zur Befüllung des Fluidspeichers (4) der Vorrichtung (1), ausgebildet zur Herstellung einer lösbaren Fluidverbindung zwischen dem Fluidspeicher (4) und dem Vorratsspeicher zur Wiederbefüllung des Fluidspeichers (4) der Vorrichtung (1) aus dem Vorratsspeicher.

## Claims

1. Transportable device (1) for providing a cooled oxygen-containing gas stream (2) for delivery to a body of a mammal via the respiratory tract, in particular a human, in order to lower the body temperature,
with a storage device (3) having at least one fluid reservoir (4) and with at least one heat exchanger (10, 13),
wherein the storage device (3) is designed to store an oxygen-containing fluid (5) in the fluid reservoir (4) and to provide a withdrawal flow (6) of the oxygen-containing fluid (5) from the fluid reservoir (4),
wherein the heat exchanger (10, 13) is designed to cool the withdrawal flow (6) provided from the fluid storage device (4) by heat transfer to a cooling flow (7) of a stored fluid (5) provided by the storage device (3) before the withdrawal flow (6) is supplied to the body,
wherein a cooled, oxygen-containing gas stream (2) emerges from the heat exchanger (10, 13) for delivery to the body, and
wherein the withdrawal flow (6) is cooled by the cooling flow (7) after its exit from the fluid reservoir (4) and before its supply to the body,
**characterised in**
**that** the cooling flow (7) is formed by a bypass flow of the oxygen-containing fluid (5) stored in the fluid container (4), wherein the withdrawal flow (6) and the cooling flow (7) have the same composition and wherein a heated first mass flow of the stored oxygen-containing fluid (5) is cooled to a specific lower temperature by a second mass flow of the same oxygen-containing fluid (5) as a bypass flow, and wherein the cooled oxygen-containing gas flow (2) is obtainable by at least partially combining the withdrawal flow (6) and the cooling flow (7).

2. Transportable device (1) according to claim 1,
**characterised in**
**that** the heat exchanger (13) is designed for direct heat transfer between the extraction flow (6) and the cooling flow (7) by mixing the extraction flow (6) and the cooling flow (7).

3. Transportable device (1) according to claim 1 or 2, **characterised in that** a control and/or regulating device (12) is designed to control and/or regulate the temperature of the cooled oxygen-containing gas flow (2) by varying the magnitude of the cooling flow (7).

4. Transportable device (1) according to one of the preceding claims, **characterised in that** the fluid reservoir (4) contains a deep-cooled, liquefied oxygen-containing fluid (5), in particular liquid oxygen or liquefied air.

5. Transportable device (1) according to any one of the preceding claims, **characterised in that** the extraction stream (6) and/or the cooling stream (7) has an oxygen content of at least 20% by weight, preferably of more than 50% by weight, further preferably of more than 90% by weight.

6. Transportable device (1) according to one of the preceding claims, **characterised in that** the withdrawal flow (6) and the cooling flow (7) are provided by the storage device (3) at the same temperature level.

7. Transportable device (1) according to one of the preceding claims, **characterised in that** at least one preheater (9) arranged upstream of the heat exchanger (10, 13) in the flow direction of the extraction flow (6) is provided for preheating the extraction flow (6), the extraction flow (6) being heated in the preheater from an outlet temperature on leaving the storage device (3) to a temperature above the temperature of the cooling flow (7), in particular by heat transfer from the environment.

8. Transportable device (1) according to claim 7, **characterised in that** at least one further heat exchanger (10) is provided between the preheater (9) and the heat exchanger (13) for pre-cooling the withdrawal flow (6) by indirect heat transfer to the cooling flow (7).

9. System with at least one device (1) according to one of the preceding claims and with a stationary storage reservoir for filling the fluid reservoir (4) of the device (1), designed to establish a detachable fluid connection between the fluid reservoir (4) and the storage reservoir for refilling the fluid reservoir (4) of the device (1) from the storage reservoir.

## Revendications

1. Dispositif transportable (1) destiné à fournir un flux de gaz (2) contenant de l'oxygène et refroidi pour l'amener au corps d'un mammifère par les voies respiratoires, en particulier d'un être humain, afin d'abaisser la température du corps,
avec un dispositif de stockage (3) présentant au moins un réservoir de fluide (4) et avec au moins un échangeur de chaleur (10, 13),
le dispositif de stockage (3) étant conçu pour stocker un fluide (5) contenant de l'oxygène dans le réservoir de fluide (4) et pour fournir un flux de prélèvement (6) du fluide (5) contenant de l'oxygène dans le réservoir de fluide (4),
l'échangeur de chaleur (10, 13) étant conçu pour refroidir le courant de prélèvement (6) fourni par le réservoir de fluide (4) par transfert de chaleur à un courant de refroidissement (7) d'un fluide (5) stocké et fourni par le dispositif de stockage (3) avant l'amenée du courant de prélèvement (6) au corps,
un courant de gaz (2) refroidi et contenant de l'oxygène sortant de l'échangeur de chaleur (10, 13) pour être amené au corps et
le flux de prélèvement (6) étant refroidi par le flux de refroidissement (7) après sa sortie de l'accumulateur de fluide (4) et avant d'être amené au corps,
**caractérisé en ce que**
**en ce que** le courant de refroidissement (7) est formé par un courant de dérivation du fluide oxygéné (5) stocké dans le récipient de fluide (4), le courant de prélèvement (6) et le courant de refroidissement (7) présentant une composition identique et un premier courant massique chauffé du fluide oxygéné (5) stocké étant amené à une température déterminée avec un deuxième courant massique du même fluide oxygéné (5) en tant que courant de dérivation, température plus basse et dans lequel le courant gazeux (2) refroidi contenant de l'oxygène peut être obtenu par une réunion au moins partielle du courant de prélèvement (6) et du courant de refroidissement (7).

2. Dispositif transportable (1) selon la revendication 1,
**caractérisé en ce que**
**en ce que** l'échangeur de chaleur (13) est conçu pour le transfert de chaleur direct entre le courant de prélèvement (6) et le courant de refroidissement (7) par mélange du courant de prélèvement (6) et du courant de refroidissement (7).

3. Dispositif transportable (1) selon la revendication 1 ou 2, **caractérisé en ce qu'**il est prévu un dispositif de commande et/ou de régulation (12) conçu pour commander et/ou réguler la température du flux de gaz (2) refroidi contenant de l'oxygène en modifiant la taille du flux de refroidissement (7).

4. Dispositif transportable (1) selon l'une des revendications précédentes, **caractérisé en ce que** le réservoir de fluide (4) contient un fluide oxygéné (5) liquéfié et surgelé, notamment de l'oxygène liquide ou de l'air liquéfié.

5. Dispositif transportable (1) selon l'une des revendications précédentes, **caractérisé en ce que** le flux de prélèvement (6) et/ou le flux de refroidissement (7) présente une teneur en oxygène d'au moins 20 % en poids, de préférence supérieure à 50 % en poids, de préférence encore supérieure à 90 % en poids.

6. Dispositif transportable (1) selon l'une des revendications précédentes, **caractérisé en ce que** le flux de prélèvement (6) et le flux de refroidissement (7) sont fournis par le dispositif de stockage (3) à un même niveau de température.

7. dispositif transportable (1) selon l'une des revendications précédentes, **caractérisé en ce qu'**il est prévu au moins un préchauffeur (9) disposé en amont de l'échangeur de chaleur (10, 13) dans le sens d'écoulement du courant de prélèvement (6) pour préchauffer le courant de prélèvement (6), le courant de prélèvement (6) étant réchauffé dans le préchauffeur d'une température de sortie à la sortie du dispositif de stockage (3) à une température supérieure à la température du courant de refroidissement (7), en particulier par transfert de chaleur depuis l'environnement.

8. Dispositif transportable (1) selon la revendication 7, **caractérisé en ce qu'**entre le préchauffeur (9) et l'échangeur de chaleur (13) est prévu au moins un autre échangeur de chaleur (10) pour le prérefroidissement du courant de prélèvement (6) par transfert indirect de chaleur au courant de refroidissement (7).

9. Système comprenant au moins un dispositif (1) selon l'une des revendications précédentes et comprenant un réservoir disposé de manière stationnaire pour remplir le réservoir de fluide (4) du dispositif (1), conçu pour établir une liaison fluidique amovible entre le réservoir de fluide (4) et le réservoir pour remplir à nouveau le réservoir de fluide (4) du dispositif (1) à partir du réservoir.
